# EUROPEAN PATENT SPECIFICATION

(11) **EP 3 229 861 B1**
(45) Date of publication and mention of the grant of the patent: **30.01.2019**
(21) Application number: 15808210.7
(22) Date of filing: 11.12.2015
(51) Int. Cl.: A61M 5/00

(54) **MEDICAL APPARATUS PACKAGE**
MEDIZINISCHE VORRICHTUNGSPACKUNG
BOÎTIER D'APPAREIL MÉDICAL

(30) Priority: 12.12.2014 EP 14307014
(43) Date of publication of application: 18.10.2017
(73) Proprietor: SANOFI, 75008 Paris (FR)
(72) Inventor: DITTRICH, Marcus-Meinolf, 65926 Frankfurt (DE)
(74) Representative: Brown, Alexander Edward
(86) International application number: PCT/EP2015/079364
(87) International publication number: WO 2016/092058

(56) References cited:
- WO-A1-2006/015117
- WO-A1-2013/043177
- GB-A- 2 394 943
- US-A1- 2009 206 092

## Description

### Field of the Invention

The invention is defined in the appended claims and relates to a package and, in particular, to a package for a medical apparatus and/or a part or parts thereof.

### Background of the Invention

A variety of diseases exist that require regular treatment by injection of a medicament. Injection devices known in the art include infusion and patch pumps for delivering injections of medicament. Another type of injection device that is gaining traction is a bolus injector. Biological medicaments are being increasingly developed which comprise higher viscosity injectable liquids and which are administered in larger volumes than traditional liquid medicaments. Some bolus injectors are intended to be used with relatively large volumes of medicament, typically at least 1 ml and maybe a few ml. Injection of such large volumes of medicament can take some minutes or even hours. Such high capacity bolus injectors can be called large volume devices (LVDs) and may comprise a pre-filled disposable medicament delivery device or, alternatively, a disposable medicament delivery device into which a patient or medical personnel must insert a medicament cartridge prior to use. To use an injection device, such as an LVD, it is first supported on a suitable injection site of a patient and, once installed, injection is initiated by the patient or another person (a user).

Particularly in the case of patient-operated devices which require insertion of a medicament cartridge prior to use, the medicament delivery process from start to finish can be a complicated multi-step process. Steps can include gathering all of the device components, sterilization of the preparation area, removal of each component from their respective packaging, assembly of the components to ready the device for medicament administration before the actual process of injecting the medicament can even begin. An example of a package for holding and storing articles such as drug delivery devices is known from WO 2006/015117. Some medicament delivery devices are provided pre-loaded with a medicament to be administered, whereas other delivery devices comprise a chamber to receive a separate cartridge of medicament which the patient must load into the chamber of the delivery device to prime the device ready for use. Such processes are therefore complicated for the patient to remember and execute, intrusive upon his or her daily schedule, and there remains the risk that the patient may not correctly perform the medicament administration process, particularly if the patient is not familiar with the process.

Medical devices are often used by persons without particular medical and/or technical skills such as for example patients themselves. Therefore, packages for complex devices, such as for medical devices, often contain a manual with instructions for the first use in order to ensure that the steps necessary to use the device are performed correctly by the patients. However, if such a manual is placed in the package, it may be ignored by the patient. In particular, user studies showed that the first action of users is often to remove the manual and to put it aside, thereby removing it from the field of view so that it went out of the users' mind.

Studies further show that when patients are interacting for the first time with a medical device, for example an electronic medical device, they make reference to their knowledge of other electronic devices. When there are operating differences between the actual device and the mental model, however, this may result in errors in device usage. It was also found that patients can be confused by a package containing a significant amount of components to be used or assembled for operating the device as it is not evident which component has to be used first.

Accordingly, there exists a need for providing a patient with a medicament delivery apparatus and administration process that is simple to use and is not unduly burdensome on the patient's daily schedule.

### Summary of the Invention

The present invention provides a package for components of a medical apparatus comprising a package for components of a medical apparatus comprising a plurality of panels defining a container having an interior space, and a first component of the medical apparatus disposed within the interior space, the first component of the medical apparatus comprising a medicament delivery device, wherein the container comprises a base panel which forms a bottom wall of the container, and at least one lid panel which is connected to the base panel and is foldable into a position in which it extends co-planar with the base panel when the container is manipulated into an open configuration, and characterized in that the at least one lid panel includes a sterile surface forming a preparation surface upon which a user may place components of a medical apparatus when the container is in the open configuration.

The package may advantageously provide a preparation surface for components of a medical apparatus without the need for a user to sterilize, sanitize, disinfect or otherwise clean an underlying surface such as a table top or kitchen counter top. This may help towards ensuring a clean, disinfected and/or sterile area for components of the medical apparatus to be placed during use of the apparatus.

The sterile surface may comprise a coating comprising a sterilizing agent applied to a surface of the at least one lid panel. This may advantageously enable the preparation surface to remain clean, disinfected and/or sterile for longer, and may reduce the likelihood of contamination of the surface.

The at least one lid panel may be foldable into a closed configuration in which the at least one lid panel at least partially encloses the interior space.

The sterile surface of the lid panel may forms an interior surface of the container when the container is in the closed configuration. This may advantageously shield the preparation surface from external elements before use, to help avoid contamination of the surface.

The container may comprise a plurality of lid panels overlying each other in the closed position of the package, and said at least one lid panel including the preparation surface may comprise the innermost lid panel. This may further advantageously help to prevent contamination of the preparation surface.

The at least one lid panel may be bonded to the base panel in the closed configuration of the container. This may advantageously seal the package closed before use.

The at least one lid panel may be bonded to the base panel by a second component of the medical apparatus being bonded to the base panel. This may force a patient or user to handle the component before use of device, and thereby create a memory aid and/or force a procedural step of use of the apparatus.

The second component of the medical apparatus may comprise a packet containing a sterilizing agent. This may advantageously help ensure that the patient or user comes across sterilizing agent in opening package and therefore is reminded to use it at that point in the procedure. The at least one lid panel may be connected to the base panel by an intermediate panel which comprises a side wall panel of the container.

The panels of the container may be formed from a single planar blank of material. This may advantageously facilitate ease and/or simplicity of manufacture of the container.

The container may be configurable into an open configuration in which all panels of the container lie co-planar with each other. This may advantageously provide a presentable open configuration of the container for the patient or user, and may provide enhanced usability of the package with the contained medical apparatus.

At least one panel of the container may be printed with instructions for use of the medicament delivery device. This may help avoid the need for multiple components to the apparatus, and may avoid the requirement for a separate instruction manual which would require handling by a patient or user.

The container of the package may comprise a plurality of lid panels overlying each other in a closed configuration of the container, wherein said panel including the instructions for use may comprise the outermost lid panel. This may help ensure the patient or user sees instructions on first opening of package and may help to avoid errors in use of the apparatus.

The medicament delivery device may comprise a bolus injector device.

The present invention also provides a medical apparatus comprising a package as described above, further comprising a container of medicament within the interior space of the container.

### Brief Description of the Drawings

Embodiments of the invention will now be described, by way of example only, with reference to the accompanying drawings, in which:
Figure 1 shows a perspective view of a package of a first embodiment of the invention in a closed configuration;
Figure 2 shows a perspective view of the package of figure 1 in a first intermediate configuration in which the package is partially open;
Figure 3 shows a perspective view of the package of figures 1 and 2 in a second intermediate configuration in which the package is partially open; and
Figure 4 shows the package of figures 1 to 3 in an open configuration.

### Detailed Description

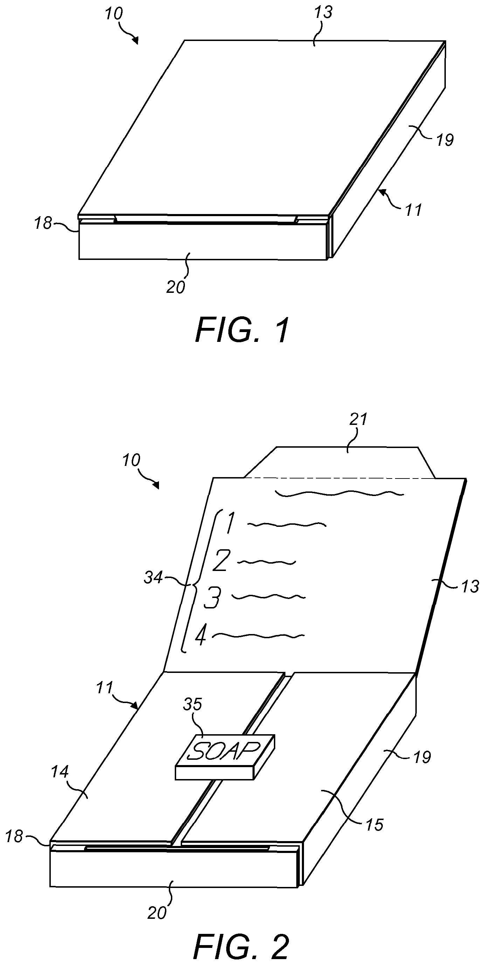

Figures 1 to 4 show a medicament package 10 according to a first embodiment of the invention which comprises a container 11 and various components of a bolus injector apparatus and associated materials held within the container 11. The container 11 is formed from folded blank of card or other suitable sheet material. The blank that forms the container 11 comprises various integrally-formed rectangular panels and includes a base panel 12, an outer lid panel 13, first and second side lid panels 14, 15 and an inner lid panel 16. The lid panels 13, 14, 15, 16 are connected to edges of the base panel 12 via respective intermediate panels (17, 18, 19, 20) which comprise wall panels of the assembled container 11. The outer lid panel 13 is joined to one edge of the base panel 12 via a first wall panel 17. The first and second side lid panels 14, 15 are joined to opposite edges of the base panel 12, adjacent the outer wall panel 17, by second and third wall panels 18, 19 respectively. The inner lid panel 16 is joined to an edge of the base panel 12 opposite the first wall panel 17 by a fourth wall panel 20.

An edge of the outer lid panel 13 remote from the first wall panel 17 includes a closure tab 21. The inner lid panel 16 includes a tab slot 22 proximate the fold line between the inner lid panel 16 and the fourth wall panel 20.

The inner lid panel 16 comprises a first flap 23 remote from the fourth wall panel 20, and a second flap 24 adjacent the first flap.

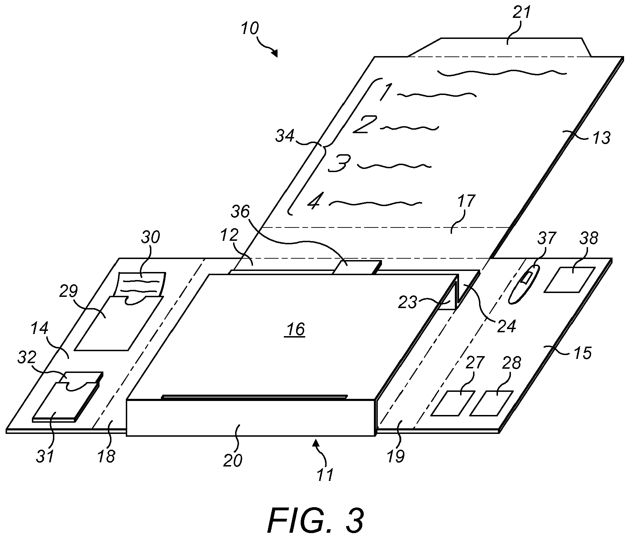

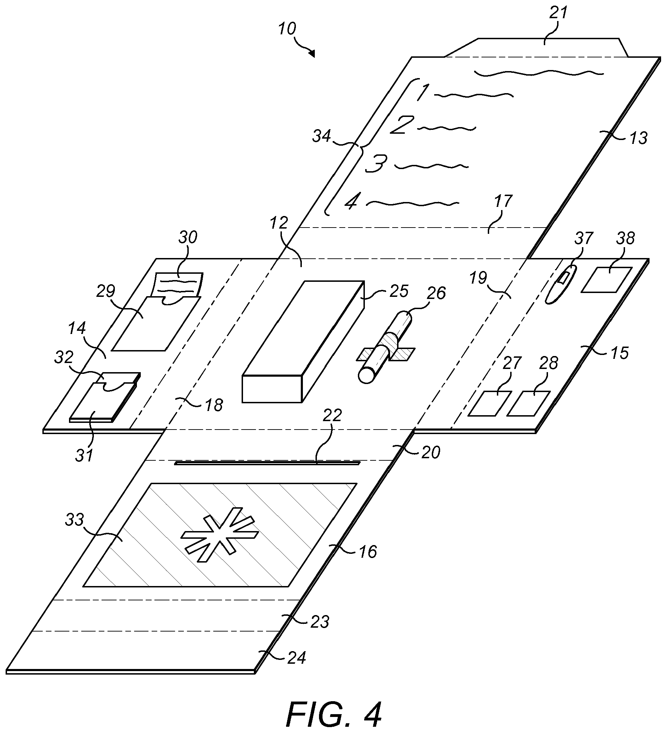

Fold lines extend across the joins between adjacent packaging panels, shown by dashed lines in figures 3 and 4.

Various components of a bolus injector apparatus and associated materials required in use of the device are contained within the container 11, and includes a bolus injector device 25 (hereafter referred to as "device 25") and a medicament cartridge 26 to be inserted into the device 25. A sterilizing swab 27 and a gauze pad 28 are provided on the second side lid panel 15. The sterilizing swab 27 may be used to disinfect the intended injection site on the patient's skin. The gauze pad 28 may be used for drying the injection site of residual sterilizing agent from the sterilizing swab 27. The first side lid panel 14 is provided with a pocket 29 containing an instruction sheet 30 to guide a patient through proper use of the device 25. The first side lid panel 14 may optionally also include a second pocket 31 containing a detailed instruction manual 32 providing more detailed instructions and specifications of the device 25 and medical/pharmacological information about the medicament within the medicament cartridge 26.

A surface of the inner lid panel 16 is configured to serve, in use of the package 10, as a preparation surface upon which a patient or other user may rest components of the bolus injector apparatus, including the device 25 and the medicament cartridge 26. The preparation surface of the inner lid panel 16 may include a marking, indicia or other graphics 33 to denote the surface of the inner lid panel 16 as a preparation surface. The surface of the inner lid panel 16 is sterile, and/or may be provided with a sterile, sterilizing or disinfectant coating or other treatment to maintain the sterility of the surface. The sterile coating may comprise a sterilizing agent within a carrier material that is applied to the preparation surface. The sterilizing agent may comprise an ink that is printed onto the preparation surface, and the marking 33 denoting the preparation surface may comprise such a sterilizing ink. The preparation surface may alternatively, or in addition, be pre-treated with a sterilizing agent during manufacture of the container blank and/or of the package 10.

A surface of the outer lid panel 13 may include high level instructions 34 denoting sequential instructions for use of the package 10, or for operation of the device 25, or other relevant information for the patient or other user.

From figure 4, it can be seen that the device 25 and the medicament cartridge 26 are provided on the base panel 12 of the container, and may be provided in a hermetically sealed blister pack (not shown) that is bonded to the base panel 12, or may be otherwise secured in place on the base panel 12. Yet further, the device 25 and the medicament cartridge 26 may not be fixedly secured to the base panel 12 but instead may simply be placed on the base panel 12 and confined within the container 11 when the container is folded into the closed configuration shown in figure 1.

In the closed configuration of the package 10 shown in figure 1, the device 25 and medicament cartridge 26 are disposed on the base panel 12 and the fourth wall panel 20 extends perpendicularly from the base panel 12. The inner lid panel 16 is in a closed position in which it extends perpendicularly from the fourth wall panel 20 to cover the device 25 and medicament cartridge 26. The inner lid panel 16 thereby forms an upper wall of the interior space of the container 11, and thus at least partially encloses the interior space. The first flap 23 of the inner lid panel 16 extends to the base panel 12 and the second flap 24 of the inner lid panel 16 lies flush with the base panel 12. This configuration can be seen in the second intermediate position shown in figure 3.

The second and third wall panels 18, 19 extend perpendicularly from the base panel 12 and the first and second side lid panels 14, 15 are folded into a closed position in which they extend perpendicularly to the second and third wall panels 18, 19 and onto the inner lid panel 16. The first and second side lid panels 14, 15 thereby form an upper wall of the interior space of the container 11, and thus at least partially enclose the interior space. This configuration can be seen in the first intermediate configuration shown in figure 2.

The first wall panel 17 extends perpendicularly to the base panel 12 and the outer lid panel 13 is folded into a closed position in which it extends perpendicularly to the first wall panel 17 and onto the first and second side lid panels 14, 15. The closure tab 21 is received in the tab slot 22 to hold the package 10 closed. The outer lid panel 13 thereby forms an upper wall of the interior space of the container 11, and thus at least partially encloses the interior space.

Referring to figure 2, a packet 35 is bonded across the first and second side lid panels 14, 15 and holds them together, preventing access to the interior of the package 10. The packet 35 contains a soap bar or other disinfecting or sterilizing agent (not shown) to enable a patient to sterilize their hands prior to handling the device 25. The packet 35 is removable to enable the first and second side lid panels to be folded away from the inner lid panel 16.

The second flap 24 is secured to the base panel 12 by a sachet 36 being bonded across the edge of the second flap 24 and across the base panel 12. The sachet 36 contains a sterilizing swab (not shown) to enable a patient to clean and sterilize an area of their skin to serve as an injection site in a subsequent medicament administration process. The sachet 36 is removable to enable the second flap 24 and thereby the whole of the inner lid panel 16 to be folded away from the base panel 12. This sachet 36 may be in addition to, or in place of, the sterilizing swab 27 on the inside of the second side lid panel 15.

Use of the package 10 of the exemplary embodiment of the invention will now be described. Use will be described in terms of a patient using the package for self-administration of a medicament using the device 25, although it should be appreciated that use of the package 10 of the invention is not limited to use by a patient but includes use by third parties on behalf of a patient, for example by medical personnel.

A patient takes the package 10 in the closed configuration and lifts the closure tab 21 from out of the tab slot 22, and folds the outer lid panel 13 away from the first and second side lid panels 14, 15. The package 10 is then in the first intermediate configuration shown in figure 2. The patient is then presented with the packet 35 containing the soap and is unable to proceed with opening the package further until the packet 35 is removed. This forces the patient to handle the packet 35 and notice the soap, and serves to remind the patient that they are required to wash their hands before proceeding to the next step in the process.

At this stage, the patient is also presented with the high-level instructions 34 on the inner face of the outer lid panel 13 and so has clear operation instructions in front of them from an early stage in the process. In an exemplary embodiment of the invention, the high level instructions 34 may read "1. Wash hands. 2. Prepare Injector. 3. Inject. 4. Dispose of Injector". In a further alternative exemplary embodiment of the invention, the high level instructions 34 may read "1. Wash hands. 2. Sterilize injection site. 3. Prepare injector. 4. Inject. 5. Dispose of Injector".

After removing the packet 35, the patient can then fold the first and second side lid panels 14, 15 away from the inner lid panel 16 from their closed position to an open position. The package 10 is then in the second intermediate configuration shown in figure 3. Here, the interior space of the container 11 remains enclosed only by the inner lid panel 16 remaining in its closed position. The patient is then presented with the instruction sheet 30 and detailed instruction manual 32, if required, providing information on correct use of the device 25. The user is also presented with the sachet 36 containing the sterilizing swab and is unable to proceed with opening the package further until the sachet 36 is removed. This forces the patient to handle the sachet 36 and notice the sterilizing swab, and serves to remind the patient that they are required to sterilize the intended injection site on their skin before proceeding to the next step in the process and before access to the device 25 is possible.

Having removed the sachet 36 and sterilized the injection site, the patient is then able to unfold the inner lid panel 16 away from the base panel 12. The inner lid panel 16 is unfolded from its closed position to an open position. The inner lid panel 16 can be folded flat, parallel and co-planar with the base panel 12. The package 10 is then in the open configuration shown in figure 4. This leaves the apparatus preparation surface facing upwards. The graphics 33 denoting the preparation surface are exposed on the surface of the inner lid panel 16 as a visual indicator to the patient. The patient is then able to use the preparation surface of the inner lid panel 16 to place the device 25 or medicament cartridge 26 on, without contaminating either from contact with other surfaces.

The patient is then presented with the device 25 and medicament cartridge 26. The patient can then load the medicament cartridge 26 into the device and can use the device 25 as per the manufacturer's instructions to perform a medicament administration process. After use of the device 25, the device 25 and medicament cartridge 26 may be discarded. Alternatively, the medicament cartridge 26 may be removed and discarded and the device 25 may be retained for future use, if it is a reusable device.

The second side lid panel 15 may also include materials that may be optionally required during or after a medicament delivery process. This may include a plaster 37 and/or an additional gauze pad 38.

The package 10 including a device preparation surface, formed by the inner lid panel 16 folding out flat, advantageously may avoid the need for a patient or other user to clean and/or disinfect an underlying surface upon which the package 10 is placed during use. For example, it may not be necessary to sterilize an area on a table surface before initiating use of the package 10. This may help minimize the risk of contamination of the components of the medicament apparatus. This may also make use of the package 10, and the whole medicament administration process, simpler for the patient and less of an onerous exercise. Furthermore, the preparation surface being sterile avoids the need for an additional surface sterilizing step by the patient. The preparation surface being sterile also may help ensure that there is no contamination of the preparation surface or any medical apparatus components placed thereon during the medicament administration process.

The preparation surface of the inner lid panel 16 forms an interior surface of the container 11 when the container 11 is in the closed configuration. That is, the preparation surface faces inwardly when the container 11 is in the closed configuration. This may advantageously help avoid exposure of the preparation surface to environmental elements, and therefore avoid contamination of the preparation surface before use of the package 10. The preparation surface is also formed on the innermost lid panel of the container 11. That is, in the closed configuration of the container 11, the inner lid panel 16 is innermost, the outer lid panel 13 is outermost, and the first and second side lid panels 14, 15 are disposed between the outer lid panel 13 and the inner lid panel 16. There is therefore at least one further panel of the container 11 that lies over the inner lid panel 16 and its associated preparation surface preventing its direct exposure to ambient conditions. This may further advantageously shield the inner lid panel 16 and its preparation surface from environmental elements during transport and storage of the package 10 and before use. This may provide the above-described benefit of retaining the sterility of the preparation surface.

The package 10 of the invention including all necessary materials required for a patient to complete a medicament delivery process helps make the process easy to follow, and avoids the need for the patient to gather materials from various sources prior to beginning the medicament administration process. This may help avoid patient errors in the administration procedure, and also is reassuring for the patient, helping them be more relaxed and make the process less intrusive upon his or her lifestyle.

Although the package 10 is described as including a device 25 and a separate medicament cartridge 26, the invention is not limited to this configuration and a package of the invention may alternatively include a device 25 pre-loaded with medicament. Such a device 25 may include a pre-loaded cartridge 26 or may include a reservoir integrally formed with the device 25. Furthermore, the invention is not intended to be limited to a package 10 in which the device 25 receives a medicament cartridge 26. The device 25 may receive any form of medicament container or reservoir within the scope of the invention, and be configured to dispense medicament from such container or reservoir to a patient.

The package 10 of the exemplary embodiment is described as having the packet 35 and sachet 36 which are bonded in place to prevent a patient from opening the package 10 further until each is detached from the package 10. However, although each serves as a process reminder to the patient, the invention is not intended to be limited to either of these components being bonded in place in the package 10. The package may alternatively include an alternative component of the medical apparatus bonded in place to retain one or both of the inner lid panel 16 and first and second side lid panels 14, 15 in their closed position. One or both of the packet 35 and sachet 36 may be provided elsewhere in the package that does not necessarily require removal for complete opening of the package 10. Yet further, the package 10 may include a sealing label in place of the packet 35 and/or sachet 36 to perform the function of retaining the pack in the partially-closed, first and second intermediate configurations. Alternatively, the relevant panels/tabs could be adhered together in an area of overlap to retain the package 10 in these configurations. This may be the case particularly if the packet 35 of soap and/or sachet 36 of sterilizing swab are provided elsewhere within the package 10. If the package 10 includes a label in place of the packet 35 and/or sachet 36, the label may include text and/or graphics to remind a patient or other uses to wash their hands or sterilize an intended injection site. Therefore, the function of a process reminder before enabling opening of the package 10 may be retained without the patient actually handling the soap packet 35 or sterilizing swab sachet 36.

The particular configuration of medicament package 10 shown and described above is exemplary only and only serves to illustrate the inventive concept of the medicament package of the invention. It should be understood that alternative configurations of package and container are intended to be encompassed within the scope of the invention. For example, one or more panels of the container may be rectangular, square, or any other suitable shape. It will also be appreciated that other types of medicament delivery device and medicament container may be used with the package 10 within the scope of the invention. The package 10 may be used with both liquid and gaseous medicament and associated devices. For example, the medicament may comprise a gaseous inhalable medicament. Such a medicament may be provided in a pressurized medicament container such as a canister which is receivable in a medicament delivery device.

The container 11 of the package 10 may be made from a blank of card, although the invention is not limited to such material and any other suitable material may be used within the scope of the invention, including plastic or metal. The container 11 may be made from a hybrid of materials, such as plastic laminated card. The container 11 may be folded from a single blank of material. The fold lines may comprise lines of weakness in the sheet material of the container 11. The lines of weakness may be lines of reduced thickness or other deformation in the blank. The lines of weakening may include one or more score lines, perforations, or embossing of the material of the body 11. This may be advantageous as being a cost-effective and easily manufactured configuration of package container.

The container may be formed from a single unitary blank of material. However, the invention is not intended to be limited to this configuration and the container 11 may be formed from a plurality of packaging components connected together. Various panels may be separate components connected together. The panels may be connected by sections of material which may be a different material to that of the panels. For example, the connecting material may be a more flexible material than that of the panels. For example, the panels may be made from stiff card and the sections of connecting material may be flexible plastic or rubber, or more flexible card.

The package 10 of the invention is not intended to be limited to a container formed from flat blank(s) of material, and instead one or more parts of the package may be formed as a nonplanar structure. For example, the base panel 12 may be replaced by a tray-shaped container with the first and second side lid panels 14, 15 and outer lid panel 13 connected to the tray-shaped container. However, in such an embodiment, a panel of the package would still need to be foldable flat and co-planar with the base surface of the tray-shaped container. As such, the inner lid panel 16 may still be connected to a base surface of such a tray-shaped container by a fourth panel 20 so as to be foldable as described previously.
Although the device 25 and the medicament cartridge 26 are described above as optionally being provided in a hermetically sealed blister pack that is bonded to the base panel 12, other means for securing the device 25 and medicament cartridge 26 in place are intended to fall within the scope of the invention. For example, the device 25 and/or the medicament cartridge 26 may be retained by surface formations formed in the base panel 12. For example, the base panel 12 may be formed with projections which secure the device 25 and/or the medicament cartridge 26 as a friction fit or snap-fit in place on the base panel 12. Such formations may be embossed or punched in the sheet material from which the container 11 is formed. Such mechanical securing of the device 25 and/or the medicament cartridge 26 may be in addition to, or instead of, use of an adhesive to bond the components in place.

The package 10 may not include separate side lid panels 14, 15 and may instead include one single side lid panel that extends partially or entirely over the inner lid panel 16. Such configuration would still serve to prevent opening of the inner lid panel 16 until the side lid panel had been folded out. As such, the single side lid panel may be bonded closed at an edge of the side lid panel and the inner lid panel 16. The bonded closure may be by a packet 35 or label overlying the side lid panel edge and the inner lid panel 16. Alternatively, adhesive may be applied between the side lid panel edge and the inner lid panel 16.

The invention is not intended to be limited to exemplary configuration of medicament package 10 shown and described above. In an alternative package within the scope of the invention, the first and second side panels 14, 15 may be first folded into their closed position and the inner lid panel 16 may be folded into its closed position over the first and second side panels 14, 15. In such a configuration, the first and second side panels 14, 15 would be disposed innermost in the container 11. It will be appreciated that in such an alternative configuration of package, the sterile preparation surface of the inner lid panel 16 would still be shielded from contamination from environmental elements, by being in contact with the first and second side lid panels 14, 15, and also by being covered by the outer lid panel 13.

In embodiments of the invention, only the inner lid panel 16 may be held closed, for example by a sachet 36 bonded across an edge thereof. Alternatively, only the first and second side panels 14, 15 may be held closed, for example by a packet 35 being bonded across them. Alternatively, both the inner lid panel 16 and the first and second side panels 14, 15 may be held closed, as described above.

The invention is not intended to be limited to an embodiment in which the inner lid panel 16 which provides the preparation surface extends across the entire width of depth of the container 11. In alternative embodiments, the inner lid panel 16 may be narrower or shallower than shown and described above so that is only partially extends across the width or depth of the container 11 in its closed position. Such a reduced size of inner lid panel may advantageously reduce material cost of the package 10. However, it may be advantageous to maximize the side of the inner lid panel 16 to maximize the size of a preparation surface the package 10 can provide.

Embodiments of the invention may omit the outer lid panel 13 and/or the first and second side lid panels 14, 15, such that the inner lid panel 16, that comprises the preparation surface, comprises the sole lid panel of the package 10.

The sterilizing swab provided in the sachet 36 and/or the sterilizing agent within the packet 35 may comprise an absorbent pad impregnated with a suitable sterilizing agent. The term "sterilizing agent" is intended to encompass sterilizing chemicals and components or materials comprising a sterilizing chemical, including swabs, wipes, bars, gels and liquids. Similarly, the sterile preparation surface of the inner lid panel may be pre-treated with, or comprise a material impregnated with any suitable sterilizing agent or chemical. Exemplary sterilizing agents include, but are not limited to, isopropanol, isopropyl alcohol, isopropyl alcohol as a dissolution, for example isopropyl alcohol as a 70% dissolution, tincture of ionide, hydrogen peroxide, chloramine T, alcohol (e.g. ethanol, 1 - propanol), phenols, nitrogen compounds, chlorhexidin, and/or detergents. Furthermore, use of the term "sterile", "sterilizing" and "sterilized" throughout the specification and in the context of the present invention is intended to also encompass surfaces, agents and actions that are cleaned, disinfected and/or sanitized. The term "sterile" may be used interchangeably with the terms "cleaned", "disinfected" and/or "sanitized". That is, the term "sterile" is intended to encompass varying degrees of enhanced cleanliness in which the presence of germs, bacteria, microorganisms and/or other contaminants is reduced to reduce the risk patient infection during use of the package and medical apparatus of the invention. The term "sterile" is not intended to be limited to a definition of a complete absence, destruction and/or removal of such contaminants.

The gauze pad(s) 28, 38 may comprise any suitable material and may include, but are not limited to, gauze sponge, cotton, cellulose, rayon, or other porous filter material. Furthermore, the package 10 of the invention is not limited to embodiments in which a gauze pad 28, 38 is provided and alternative embodiments of the invention may omit the gauze pads 28, 38.

The device 25 may be configured to deliver the medicament subcutaneously, although it may instead be configured for intradermal injection, for instance using a microneedle, or for injection in some other manner.

The device 25 may be a bolus injector device of the type known as a Large Volume Device (LVD). An LVD injection device is configured to dispense a relatively large dose of medicament, in particular at least 1 ml and typically up to 2.5 ml, but possibly up to 10 ml.

The bolus injector device is configured to deliver a bolus of the respective medicament to bring a volume of the medicament into a patient's body within a predetermined time. The injection rate, however, may not be critical, i.e. tight control may not be necessary. However, there may be an upper (physiological) limit to the delivery rate in order to avoid damage to the tissue surrounding the delivery site. The time taken to deliver a bolus dose of medicament may be between a few minutes and many hours depending on a number of factors including the quantity (volume) of medicament, the viscosity of the medicament and the nature of the injection site at which the injection device is intended to be used.

From a user or Health Care Professional perspective, it is desirable for an injection device to be configured to minimally impact the patient's lifestyle and schedule, providing the patient with minimal reminder of his or her disease between the injections. The treatment schedule for therapies is usually intermittent, i.e. may be one injection per week, one injection every other week, or one per month. Therefore, the patient usually has no routine in dealing with his or her disease, and hence has minimal routine/experience in performing the required injections. Thus, configuration of the injection device and all associated apparatus and packaging to simplify its operation by patients is highly desirable.

Because it is intended for bolus operation, the configuration of the injection device is quite different compared to an injection device that is intended to be used for basal operation. Also, its use is quite different. For instance, a basal type insulin pump generally is relatively expensive as it includes many sophisticated diabetes specific features like programmable delivery rate profiles, bolus calculators etc. Further, the connection to the body via an infusion set allows the patient to handle and manipulate the pump in his/her field of view while the therapy is ongoing. Further, diabetes patients usually have a routine in setting-up the infusion set, connecting and operating the pump, and disconnecting the pump temporarily for events like taking a shower so not to expose the pump to water. In contrast, the bolus injector devices described above can be relatively simple and inexpensive devices. They may be provided as single-use devices, which cannot be recharged with medicament, which further reduces complexity and cost.

The term "drug" or "medicament", as used herein, means a pharmaceutical formulation containing at least one pharmaceutically active compound. In some embodiments, the pharmaceutically active compound can have a molecular weight up to 1500 Da or may include a peptide, a protein, a polysaccharide, a vaccine, a DNA molecule, an RNA molecule, an enzyme, an antibody or a fragment thereof, a hormone or an oligonucleotide, or a mixture of the above-mentioned pharmaceutically active compound. Various types or subtypes of compounds are also contemplated. For example, RNA may include RNAi, siRNA, or miRNA. In other embodiments, the pharmaceutically active compound can be useful for the treatment or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis or rheumatoid arthritis. In some embodiments, the pharmaceutically active compound can comprise at least one peptide for the treatment or prophylaxis of diabetes mellitus or complications associated with diabetes mellitus such as diabetic retinopathy. The pharmaceutically active compound can also comprise at least one human insulin or a human insulin analogue or derivative, glucagon-like peptide (GLP-1) or an analogue or derivative thereof, or exendin-3 or exendin-4 or an analogue or derivative of exendin-3 or exendin-4 or a pharmaceutically acceptable salt or solvate thereof.

Insulin analogues can include, for example, Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivatives can include, for example, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl-ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin.

Exendin-4 can include, for example, Exendin-4(1-39).

Hormones can include, for example, hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, or Goserelin.

A polysaccharide can include, for example, a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies can include generally globular plasma proteins (-150 kDa) that are also known as immunoglobulins which share a basic structure. As they can have sugar chains added to amino acid residues, they may also be classified as glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that can include four polypeptide chains; two heavy chains and two light chains connected by disulfide bonds between cysteine residues. Each heavy chain can be about 440 amino acids long; each light chain can be about 220 amino acids long. Heavy and light chains may each contain intra-chain disulfide bonds which stabilize their folding. Each chain is composed of structural domains called Ig domains. These domains typically contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of antibodies can be similar, the unique property of a given antibody is determined by the variable (V) regions, as detailed above. More specifically, variable loops, often three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is usually the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their inter-chain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H inter-chain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCI or HBr salts. Basic salts are e.g. salts having a cation selected from alkali or alkaline, e.g. Na+, or K+, or Ca2+, or an ammonium ion. Pharmaceutically acceptable solvates are for example hydrates.

In some embodiments, medicaments of various viscosities can be injected. For example, viscosity could range from about 3 to about 50 cP. In other embodiments, viscosity could be less than about 3 cP or greater than about 50 cP. Injection can further include delivering a medicament to a sub-cutaneous, an intra-muscular, or a transdermal location within a patient's body. The medicament can be in the form of a liquid, gel, slurry, suspension, particle, powder, or other type.

Typical injection volumes can range from about 1 mL to about 10 mL. Rates of injection may be about 0.5 mL/min, about 0.2 mL/min, or about 0.1 mL/min. Such injection profiles may be generally constant in flow rate, generally continuous in duration, or both generally constant and generally continuous. These injections can also occur in a single step of administration. Such injection profiles may be referred to as bolus injections.

Delivery devices functioning with such medicaments may utilize a needle, cannula, or other injection element configured to deliver a medicament to the patient. Such an injection element may, for example, have an external size or diameter of 27G or less. Further, the injection element could be rigid, flexible, and formed using a range of one or more materials. And in some embodiments, the injection element may include two or more components. For example, a rigid trocar may operate in conjunction with a flexible cannula. Initially, both the trocar and cannula may move together to pierce the skin. The trocar may then retract while the cannula remains at least partially within the target tissue. Later, the cannula may separately retract into the delivery device.

## Claims

1. A package (10) for components of a medical apparatus comprising:
a plurality of panels (12 - 20) defining a container (11) having an interior space; and
a first component of the medical apparatus disposed within the interior space, the first component of the medical apparatus comprising a medicament delivery device (25);
wherein the container (11) comprises a base panel (12) which forms a bottom wall of the container (11); and
at least one lid panel (16) which is connected to the base panel (12) and is foldable into a position in which it extends co-planar with the base panel (12) when the container (11) is manipulated into an open configuration; and
**characterized in that** the at least one lid panel (16) includes a sterile surface forming a preparation surface upon which a user may place components of a medical apparatus when the container (11) is in the open configuration..

2. A package (10) according to claim 1 wherein the sterile surface comprises a coating comprising a sterilizing agent applied to a surface of the at least one lid panel (16).

3. A package (10) according to claim 1 or claim 2 wherein the at least one lid panel (16) is foldable into a closed configuration in which the at least one lid panel (16) at least partially encloses the interior space.

4. A package (10) according to claim 3 wherein the sterile surface of the lid panel (16) forms an interior surface of the container (11) when the container (11) is in the closed configuration.

5. A package (10) according to claim 4 comprising a plurality of lid panels (13, 14, 15, 16) overlying each other in the closed position of the package (10), wherein said at least one lid panel (16) including the preparation surface comprises the innermost lid panel.

6. A package (10) according to any of claims 3 to 5 wherein the at least one lid panel (16) is bonded to the base panel (12) in the closed configuration of the container(11).

7. A package (10) according to claim 6 wherein the at least one lid panel (16) is bonded to the base panel (12) by a second component (36) of the medical apparatus being bonded to the base panel (12).

8. A package (10) according to claim 7 wherein the second component of the medical apparatus comprises a packet (36) containing a sterilizing agent.

9. A package (10) according to any preceding claim wherein the at least one lid panel (16) is connected to the base panel (12) by an intermediate panel (20) which comprises a side wall panel of the container (11).

10. A package (10) according to any preceding claim wherein the panels (12 - 20) of the container (11) are formed from a single planar blank of material.

11. A package (10) according to any preceding claim in which the container (11) is configurable into an open configuration in which all panels (12 - 20) of the container (11) lie co-planar with each other.

12. A package (10) according to any preceding claim wherein at least one panel (13) of the container (11) is printed with instructions for use of the medicament delivery device (25).

13. A package (10) according to claim 12 comprising a plurality of lid panels (13, 14, 15, 16) overlying each other in a closed configuration of the container (11), wherein said panel including the instructions for use comprises the outermost lid panel (13).

14. A package (10) according to any preceding claim wherein the medicament delivery device (25) comprises a bolus injector device.

15. A medical apparatus comprising a package (10) according to any preceding claim further comprising a container of medicament (26) within the interior space of the container (11).

## Patentansprüche

1. Packung (10) für Komponenten einer medizinischen Vorrichtung, umfassend:
eine Vielzahl von Feldern (12 - 20), die einen Behälter (11) mit einem Innenraum definieren,
und
eine erste Komponente der medizinischen Vorrichtung, die im Innenraum angeordnet ist, wobei die erste Komponente der medizinischen Vorrichtung eine Medikamenten-Verabreichungsvorrichtung (25) umfasst,
wobei der Behälter (11) ein Basisfeld (12) umfasst, das eine Bodenwand des Behälters (11) bildet, und
mindestens ein Deckelfeld (16), das mit dem Basisfeld (12) verbunden und in eine Position faltbar ist, in der es sich koplanar mit dem Basisfeld (12) erstreckt, wenn der Behälter (11) in eine offene Konfiguration manipuliert wird, und
**dadurch gekennzeichnet, dass** das mindestens eine Deckelfeld (16) eine sterile Fläche aufweist, die eine Vorbereitungsfläche bildet, auf der ein Benutzer Komponenten einer medizinischen Vorrichtung platzieren kann, wenn der Behälter (11) in der offenen Konfiguration ist.

2. Packung (10) nach Anspruch 1, wobei die sterile Oberfläche eine Beschichtung umfasst, die ein auf eine Fläche des mindestens einen Deckelfelds (16) aufgebrachtes Sterilisationsmittel umfasst.

3. Packung (10) nach Anspruch 1 oder Anspruch 2, wobei das mindestens eine Deckelfeld (16) in eine geschlossene Konfiguration faltbar ist, in der das mindestens eine Deckelfeld (16) den Innenraum mindestens teilweise umschließt.

4. Packung (10) nach Anspruch 3, wobei die sterile Fläche des Deckelfelds (16) eine Innenfläche des Behälters (11) bildet, wenn der Behälter (11) in der geschlossenen Konfiguration ist.

5. Packung (10) nach Anspruch 4, umfassend eine Vielzahl von Deckelfeldern (13, 14, 15, 16), die in der geschlossenen Position der Packung (10) übereinanderliegen, wobei das mindestens eine die Vorbereitungsfläche aufweisende Deckelfeld (16) das innerste Deckelfeld umfasst.

6. Packung (10) nach einem der Ansprüche 3 bis 5, wobei das mindestens eine Deckelfeld (16) in der geschlossenen Konfiguration des Behälters (11) an das Basisfeld (12) gebondet ist.

7. Packung (10) nach Anspruch 6, wobei das mindestens eine Deckelfeld (16) dadurch an das Basisfeld (12) gebondet ist, dass eine zweite Komponente (36) der medizinischen Vorrichtung an das Basisfeld (12) gebondet ist.

8. Packung (10) nach Anspruch 7, wobei die zweite Komponente der medizinischen Vorrichtung ein Paket (36) umfasst, das ein Sterilisationsmittel enthält.

9. Packung (10) nach einem der vorhergehenden Ansprüche, wobei das mindestens eine Deckelfeld (16) über ein Zwischenfeld (20), das ein Seitenwandfeld des Behälters (11) umfasst, mit dem Basisfeld (12) verbunden ist.

10. Packung (10) nach einem der vorhergehenden Ansprüche, wobei die Felder (12 - 20) des Behälters (11) aus einem einzigen ebenen Materialzuschnitt gebildet sind.

11. Packung (10) nach einem der vorhergehenden Ansprüche, wobei der Behälter (11) in eine offene Konfiguration konfigurierbar ist, in der alle Felder (12 - 20) des Behälters (11) koplanar zueinander liegen.

12. Packung (10) nach einem der vorhergehenden Ansprüche, wobei mindestens ein Feld (13) des Behälters (11) mit Anweisungen zur Benutzung der Medikamenten-Verabreichungsvorrichtung (25) bedruckt ist.

13. Packung (10) nach Anspruch 12, umfassend eine Vielzahl von Deckelfeldern (13, 14, 15, 16), die in einer geschlossenen Konfiguration des Behälters (11) übereinanderliegen, wobei das die Anweisungen zur Benutzung aufweisende Feld das äußerste Deckelfeld (13) umfasst.

14. Packung (10) nach einem der vorhergehenden Ansprüche, wobei die Medikamenten-Verabreichungsvorrichtung (25) einen Bolusinjektor umfasst.

15. Medizinische Vorrichtung, umfassend eine Packung (10) nach einem der vorhergehenden Ansprüche, ferner umfassend einen Behälter eines Medikaments (26) im Innenraum des Behälters (11).

## Revendications

1. Boîtier (10) pour des composants d'un appareil médical, comprenant :
une pluralité de panneaux (12-20) définissant un contenant (11) ayant un espace intérieur ; et
un premier composant de l'appareil médical disposé à l'intérieur de l'espace intérieur, le premier composant de l'appareil médical comprenant un dispositif d'administration de médicament (25) ;
le contenant (11) comprenant un panneau de base (12) qui forme une paroi inférieure du contenant (11) ; et
au moins un panneau de couvercle (16) qui est raccordé au panneau de base (12) et qui peut être replié dans une position dans laquelle il s'étend de manière coplanaire avec le panneau de base (12) lorsque le contenant (11) est manipulé dans une configuration ouverte ; et
**caractérisé en ce que** l'au moins un panneau de couvercle (16) comporte une surface stérile formant une surface de préparation sur laquelle un utilisateur peut placer des composants d'un appareil médical lorsque le contenant (11) est dans la configuration ouverte.

2. Boîtier (10) selon la revendication 1, dans lequel la surface stérile comprend un revêtement comprenant un agent de stérilisation appliqué sur une surface de l'au moins un panneau de couvercle (16) .

3. Boîtier (10) selon la revendication 1 ou la revendication 2, dans lequel l'au moins un panneau de couvercle (16) peut être replié dans une configuration fermée dans laquelle l'au moins un panneau de couvercle (16) renferme au moins en partie l'espace intérieur.

4. Boîtier (10) selon la revendication 3, dans lequel la surface stérile du panneau de couvercle (16) forme une surface intérieure du contenant (11) lorsque le contenant (11) est dans la configuration fermée.

5. Boîtier (10) selon la revendication 4, comprenant une pluralité de panneaux de couvercle (13, 14, 15, 16) se chevauchant les uns les autres dans la position fermée du boîtier (10), ledit au moins un panneau de couvercle (16) avec la surface de préparation comprenant le panneau de couvercle le plus à l'intérieur.

6. Boîtier (10) selon l'une quelconque des revendications 3 à 5, dans lequel l'au moins un panneau de couvercle (16) est collé au panneau de base (12) dans la configuration fermée du contenant (11).

7. Boîtier (10) selon la revendication 6, dans lequel l'au moins un panneau de couvercle (16) est collé au panneau de base (12) par un deuxième composant (36) de l'appareil médical qui est collé au panneau de base (12).

8. Boîtier (10) selon la revendication 7, dans lequel le deuxième composant de l'appareil médical comprend un paquet (36) contenant un agent de stérilisation.

9. Boîtier (10) selon l'une quelconque des revendications précédentes, dans lequel l'au moins un panneau de couvercle (16) est raccordé au panneau de base (12) par un panneau intermédiaire (20) qui comprend un panneau de paroi latérale du contenant (11).

10. Boîtier (10) selon l'une quelconque des revendications précédentes, dans lequel les panneaux (12-20) du contenant (11) sont formés d'une ébauche de matériau plane unique.

11. Boîtier (10) selon l'une quelconque des revendications précédentes, dans lequel le contenant (11) peut être configuré dans une configuration ouverte dans laquelle tous les panneaux (12-20) du contenant (11) sont orientés de manière coplanaire les uns avec les autres.

12. Boîtier (10) selon l'une quelconque des revendications précédentes, dans lequel au moins un panneau (13) du contenant (11) est imprimé avec des instructions d'utilisation du dispositif d'administration de médicament (25).

13. Boîtier (10) selon la revendication 12, comprenant une pluralité de panneaux de couvercle (13, 14, 15, 16) se chevauchant les uns les autres dans une configuration fermée du contenant (11), ledit panneau avec les instructions d'utilisation comprenant le panneau de couvercle le plus à l'extérieur (13).

14. Boîtier (10) selon l'une quelconque des revendications précédentes, dans lequel le dispositif d'administration de médicament (25) comprend un dispositif d'injection de bolus.

15. Appareil médical comprenant un boîtier (10) selon l'une quelconque des revendications précédentes, comprenant en outre un contenant de médicament (26) à l'intérieur de l'espace intérieur du contenant (11).
